(19) Europäisches Patentamt
European Patent Office
Office européen des brevets

(11) Veröffentlichungsnummer: **0 104 389**
**B1**

(12) # EUROPÄISCHE PATENTSCHRIFT

(45) Veröffentlichungstag der Patentschrift: **16.07.86**

(21) Anmeldenummer: **83107938.9**

(22) Anmeldetag: **11.08.83**

(51) Int. Cl.⁴: **C 07 D 215/48**, C 07 D 215/54, C 07 D 215/18, C 07 D 401/12, C 07 F 9/60, A 01 N 43/42, A 01 N 57/34

(54) **Chinolinderivate, Verfahren zu ihrer Herstellung und ihre Verwendung zur Bekämpfung unerwünschten Pflanzenwuchses.**

(30) Priorität: **07.09.82 DE 3233089**

(43) Veröffentlichungstag der Anmeldung: **04.04.84 Patentblatt 84/14**

(45) Bekanntmachung des Hinweises auf die Patenterteilung: **16.07.86 Patentblatt 86/29**

(84) Benannte Vertragsstaaten: **AT BE CH DE FR GB IT LI NL SE**

(73) Patentinhaber: **BASF Aktiengesellschaft, Carl-Bosch-Strasse 38, D-6700 Ludwigshafen (DE)**

(72) Erfinder: **Hagen, Helmut, Dr., Max-Slevogt-Strasse 17e, D-6710 Frankenthal (DE)**
Erfinder: **Kohler, Rolf-Dieter, Dr., Amselweg 3, D-6803 Edingen-Neckarhausen (DE)**
Erfinder: **Markert, Juergen, Dr., Am Speyerweg 26, D-6704 Mutterstadt (DE)**
Erfinder: **Wuerzer, Bruno, Dr., Dipl.-Landwirt, Ruedigerstrasse 13, D-6701 Otterstadt (DE)**

(56) Entgegenhaltungen:
**EP - A - 0 060 429**
**AT - A - 359 497**
**GB - A - 1 202 110**
**GB - A - 1 424 359**
**GB - A - 1 482 984**
**GB - A - 2 080 803**
**US - A - 2 665 203**

**JOURNAL OF THE CHEMICAL SOCIETY, 1947, Seiten 437-445, London, GB; L. BRADFORD et al.: "The skraup reaction with m-substituted anilines"**
**JOURNAL OF HETEROCYCLIC CHEMISTRY, Band 11, April 1974, Seiten 229-230, Provo, Utah, USA; D.M.**

(56) Entgegenhaltungen: (Fortsetzung)
**BAILEY et al.: "A facile displacement of nitro by halogen in a quinoline system"**

**Die Akte enthält technische Angaben, die nach dem Eingang der Anmeldung eingereicht wurden und die nicht in dieser Patentschrift enthalten sind.**

**EP 0 104 389 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft Chinolinderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Substituierte Chinoline mit herbiziden Eigenschaften sind aus DE-OS 23 22 143 und US-PS 2 661 276 bekannt.

Es wurde gefunden, dass Chinolinderivate der Formel

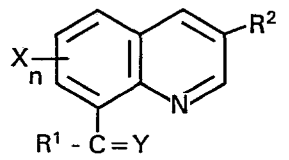

(I),

in der

X für Chlor in den Positionen 5 oder 7 steht, wobei n 1 oder 2 bedeutet,

Y für Sauerstoff oder Schwefel,

$R^1$ für Halogen, die Gruppe $-NR^3R^4$, worin $R^3$ und $R^4$ gleich oder verschieden sind und Wasserstoff, $C_{1-6}$-Alkyl, $C_{2-6}$-Alkenyl, $C_{1-6}$-Hydroxyalkyl, Cyclohexyl oder Phenyl oder $R^3$ und $R^4$ zusammen einen Tetramethylen- oder Pentamethylenrest, wobei eine $CH_2$-Gruppe durch ein Sauerstoff- oder Stickstoffatom oder die $N(CH_3)$-Gruppe ersetzt sein kann, bedeuten oder für die Gruppe OM steht, worin M ein Äquivalent eines Alkalimetall- oder Erdalkalimetallions, Wasserstoff, $C_{1-8}$-Alkyl oder den Rest $H_2N^{\oplus}R^3R^4$, in dem $R^3$ und $R^4$ die oben angegebenen Bedeutungen haben, bedeutet, und

$R^2$ für Wasserstoff, gegebenenfalls durch Halogen, Amino, Monoalkylamino, Dialkylamino oder Trialkylammonium mit 1 bis 4 Kohlenstoffatomen in einer Alkylgruppe, $C_{1-4}$-Alkoxy, $C_{1-4}$-Alkylthio, Trialkylphosphonium mit 1 bis 4 Kohlenstoffatomen in einer Alkylgruppe oder Triphenylphosphonium in ω-Stellung substituiertes $C_{1-6}$-Alkyl, für Formyl, Cyano, Carboxyl, Carbamoyl, N-Alkyl-carbamoyl oder N,N-Dialkyl-carbamoyl mit 1 bis 4 Kohlenstoffatomen in einer Alkylgruppe oder für $C_{2-6}$-Alkenyl stehen

oder in der der Rest $-\underset{\overset{\|}{Y}}{C}-R^1$ eine Nitrilgruppe oder eine Halogenmethylgruppe ist

eine herbizide Wirkung haben und sich gleichzeitig durch eine gute Verträglichkeit für bestimmte Kulturpflanzen auszeichnen.

In Formel I kann Y Sauerstoff oder Schwefel bedeuten.

$R^1$ in Formel I bedeutet Halogen, wie Brom, die Gruppe $-NR^3R^4$, in der $R^3$ und $R^4$ gleich oder verschieden sind und Wasserstoff, $C_{1-6}$-Alkyl, vorzugsweise $C_{1-4}$-Alkyl, $C_{2-6}$-Alkenyl, vorzugsweise $C_{2-6}$-Alkenyl, $C_{1-6}$-Hydroxyalkyl, vorzugsweise $C_{1-4}$-Hydroxyalkyl, Cyclohexyl oder Phenyl bedeuten oder in der $R^3$ und $R^4$ zusammen einen Tetramethylen- oder Pentamethylenrest, in denen eine $CH_2$-Gruppe durch ein Sauerstoff- oder ein Stickstoffatom oder die $N(CH_3)$-Gruppe ersetzt sein kann, bilden. Beispiele für Reste der Formel $-NR^3R^4$ sind Amino, Diethylamino, Di-ethanolamino, N-Methyl-N-n-butyl-amino, 4-Methylpiperidinyl, Cyclohexylamino, Morpholin-4-yl, Phenylamino, Pyrrolidinyl.

$R^1$ in Formel I kann ausserdem für die Gruppe OM stehen. Dabei bedeutet M Wasserstoff, ein Äquivalent eines Alkalimetall- oder Erdalkalimetallions, z.B. eines Natrium- oder Calciumions, $C_{1-6}$-Alkyl, vorzugsweise $C_{1-4}$-Alkyl oder den Rest $H_2N^{\oplus}R^3R^4$, in dem $R^3$ und $R^4$ die oben angegebenen Bedeutungen haben, vorzugsweise jedoch $C_{1-4}$-Alkyl oder $C_{1-4}$-Hydroxyalkyl, beispielsweise Methyl, Ethyl, n-Butyl, i-Butyl, s-Butyl, n-Pentyl, n-Hexyl, 2-Hydroxyethyl, bedeuten.

$R^2$ in Formel I steht für gegebenenfalls durch Halogen, Amino, Monoalkylamino, Dialkylamino oder Trialkylamino mit jeweils 1 bis 4 C-Atomen in einer Alkylgruppe, $C_{1-4}$-Alkoxy, $C_{1-4}$-Alkylthio, Trialkylphosphonium mit 1 bis 4 C-Atomen in einer Alkylgruppe oder Triphenylphosphonium in ω-Stellung substituiertes $C_{1-6}$-Alkyl, vorzugsweise $C_{1-4}$-Alkyl, insbesondere Methyl, beispielsweise Brommethyl, Dibrommethyl, Aminomethyl, Methylaminomethyl, Dimethylaminomethyl, Tetramethylammonium, Methoxymethyl, Ethoxymethyl, Methylthiomethyl, Ethylthiomethyl, Trimethylphosphoniummethyl, Triphenylphosphoniummethyl, für Cyano, Formyl, Carboxyl, Carbamoyl, N-$C_{1-4}$-Alkyl-carbamoyl oder N,N-Di-$C_{1-4}$-alkyl-carbamoyl, beispielsweise N-Methyl-carbamoyl, N,N-Dimethylcarbamoyl, oder für $C_{2-6}$-Alkenyl, vorzugsweise $C_{2-6}$-Alkenyl, beispielsweise Vinyl.

Bevorzugte Chinolinderivate der Formel I sind solche, bei denen X Chlor in 7-Stellung, n 1, $R^1$ die Gruppe OM, wobei M Wasserstoff, ein Äquivalent Alkalimetallion oder ein Dialkylammoniumion mit 1 bis 4 C-Atomen in einer Alkylgruppe bedeutet, $R^2$ $C_{1-4}$-Alkyl und Y Sauerstoff bedeuten.

Chinolinderivate der Formel I sind neu, wenn $R^2$ nicht die Bedeutung von Wasserstoff hat.

Man erhält die Chinolinderivate der Formel I durch Umsetzung von Verbindungen der Formel

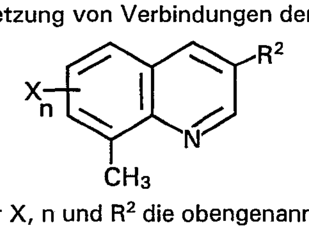

(II),

in der X, n und $R^2$ die obengenannten Bedeutungen haben, mit einem Halogenierungsmittel in Gegenwart eines Radikalstarters bei einer Temperatur zwischen 40 und 140°C und gegebenenfalls Überführung der so erhaltenen Verbindung der Formel

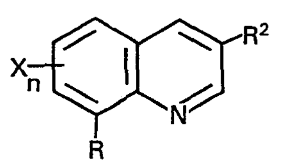

(III),

in der X, n und $R^2$ die obengenannten Bedeutungen haben und R Halogenmethyl bedeutet, in die Verbindungen der Formel I, bei denen der Rest $-\underset{\overset{\|}{Y}}{C}-R^1$ nicht für eine Halogenmethylgruppe steht.

Die Halogenierung der Verbindungen der Formel II wird zweckmässigerweise mit Halogenspendern, wie N-Chlor- bzw. N-Bromsuccinimiden, in Gegenwart von Radikalbildnern durchgeführt. Dabei erhält man Verbindungen der Formel III, in der R für Chlormethyl- bzw. Brommethyl steht. Die Halogenierung wird in einem inerten Lösungsmittel, wie z.B. Chloroform, Tetrachlorkohlenstoff oder chlorierte Benzole, in Gegenwart eines Radikalstarters, wie z.B. Azoisobutyronitril oder Benzoylperoxid, in einem Temperaturbereich zwischen 40 und 140°C, vorzugsweise 60 und 100°C, durchgeführt.

Die Überführung der Verbindungen der Formel III in Verbindungen der Formel I geschieht zweckmässigerweise in an sich üblicher Weise durch oxidative Hydrolyse in Schwefelsäure in Gegenwart von Salpetersäure.

Chinolinderivate der Formel I, in der $R^1$ für die Gruppe OM, wobei M Wasserstoff bedeutet, und Y für Sauerstoff stehen und X, n und $R^2$ die obengenannten Bedeutungen haben, erhält man durch Umsetzung einer Verbindung der Formel

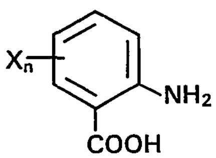

(IV),

in der X und n die obengenannten Bedeutungen haben, mit einem Aldehyd der Formel

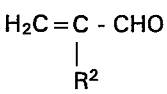

(V),

in der $R^2$ die obengenannten Bedeutungen hat.

Die Synthese von Chinolincarbonsäuren durch Skraup'sche Ringschlussreaktion von Anthranilsäuren ist bekannt [Monatsh. 2, 518 (1981). Jedoch sind die Ausbeuten speziell bei 8-Chinolincarbonsäuren häufig gering, was durch teilweise Decarboxylierung unter den Reaktionsbedingungen erklärt wird (Gazz. Chim. Ital., 16, 366 (1887)].

Deshalb war es überraschend, dass Umsetzungen von Verbindungen der Formel IV mit ungesättigten Aldehyden der Formel V die 8-Chinolincarbonsäuren der Formel I in guter Ausbeute und Reinheit ergeben. Die Umsetzung kann in Gegenwart starker Mineralsäuren, wie z.B. Salzsäure, Phosphorsäure oder Schwefelsäure, durchgeführt werden. Vorzugsweise wird Schwefelsäure mit einer Konzentration von 35 bis 95 Gew.-%, vorzugsweise 40 bis 70 Gew.-%, eingesetzt. Die Reaktionstemperaturen liegen in einem Bereich zwischen 80 und 160°C, vorzugsweise 100 und 150°C.

Chinolinderivate der Formel I, bei denen Y ein Sauerstoffatom bedeutet, erhält man durch Behandlung der entsprechenden Dichlorverbindungen mit starken Säuren, wie konzentrierte Schwefelsäure oder konzentrierte Salzsäure, bei Temperaturen zwischen 50 und 150°C.

Chinolinderivate der Formel I, bei denen Y ein Schwefelatom bedeutet, erhält man durch Umsetzen der entsprechenden Nitrile mit Schwefelwasserstoff in einem basischen Lösungsmittel, vorzugsweise Pyridin.

Die Verbindungen der Formel I, bei denen $R^1$ die Gruppe $-NR^3R^4$ bedeutet, erhält man durch Reaktion der entsprechenden Amide mit der entsprechenden Chlorverbindung bei 30 bis 100°C in An- oder Abwesenheit von Lösungsmittel, wie Alkoholen, Ethern oder Dimethylsulfoxid.

Die Verbindungen der Formel I, bei denen $R^1$ einen Rest $-OH^{\oplus}H_2R^3R^4$ bedeutet, erhält man durch Umsetzung der entsprechenden Carbonsäuren mit Aminen, beispielsweise in Alkohol, Dimethylformamid oder Dimethylsulfoxid, bei 50 bis 150°C.

Die Verbindungen der Formel I, bei denen $R^1$ für OH steht, erhält man durch Umsetzen der entsprechenden Chlorverbindungen mit Natrium- oder Kaliumhydroxid.

Chinolinderivate der Formel I, in der $R^2$ Brommethyl oder Dibrommethyl bedeutet, erhält man durch Umsetzung einer Verbindung der Formel

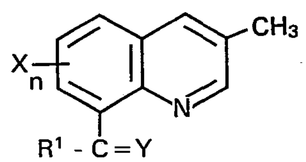

(VI),

in der X, n, $R^1$ und Y die obengenannten Bedeutungen haben, mit einem Brom liefernden Agens, z.B. N-Bromsuccinimid. Die Umsetzung wird unter den für die Halogenierung der Verbindungen der Formel II beschriebenen Bedingungen durchgeführt.

Die folgenden Beispiele erläutern die Herstellung der Chinolinderivate der Formel I.

*Beispiel 1*

8-Brommethyl-7-chlor-3-methylchinolin (Verbindung Nr. 1)

48 g 7-Chlor-3,8-dimethylchinolin, 89 g N-Bromsuccinimid und 0,5 g Azobisisobutyronitril werden in 950 g Tetrachlorkohlenstoff 24 Stunden bei 76°C gerührt. Nach dem Abkühlen wird das ausgefallene Succinimid abgesaugt und das Filtrat unter vermindertem Druck zur Trockene eingeengt. Der Rückstand wird mit Methanol aufgeschlämmt und erneut abgesaugt. Ausbeute: 30 g (56% d.Th.); Fp. 140°C (aus Ethanol).

*Beispiel 2*

7-Chlor-3-methyl-8-chinolincarbonsäure (Verbindung Nr. 2)

Zu einer Lösung von 13 g 8-Brommethyl-7-chlor-3-methylchinolin in 100 g 70%iger Schwefelsäure werden bei 100°C 10 g einer 65%igen Salpetersäure zugetropft. Nach 4stündigem Rühren bei dieser Temperatur wird die Reaktionslösung auf Eis gegeben und mit konzentrierter Natronlauge neutralisiert. Der Niederschlag wird abgesaugt, mit Wasser gewaschen und getrocknet.
Ausbeute: 5 g (45% d.Th.); Fp. 244°C (aus Dimethylformamid).

*Beispiel 3*

7-Chlor-3-methyl-8-chinolincarbonsäure
(Verbindung Nr. 2)

Zu einer Mischung aus 17 g 6-Chlor-anthranilsäure und 19 g Natrium-m-nitrobenzolsulfonat in 100 g 57%iger Schwefelsäure werden bei 100°C 14 g Methacrolein zugetropft. Nach Zugabe wird 1 Std. bei 130°C gerührt, anschliessend in 450 g Wasser eingetragen und abgesaugt. Das Filtrat wird unter Kühlung mit konzentrierter Natronlauge auf pH 2 bis 3 eingestellt. Der ausgefallene Feststoff wird abgesaugt, mit Wasser gewaschen und getrocknet.
Ausbeute: 16 g (72% d.Th.); Fp. 244°C (aus Dimethylformamid).

*Beispiel 4*

7-Chlor-3-ethyl-8-chinolincarbonsäure
(Verbindung Nr. 3)

16,8 g Ethylacrolein werden bei 100°C zu einer Lösung von 17 g 6-Chlor-anthranilsäure und 19 g Natrium-m-nitrobenzolsulfonat in 200 g 57%iger Schwefelsäure getropft. Nach 1stündigem Rühren bei 130°C wird die Reaktionslösung mit 450 g Wasser verdünnt und unter Kühlung mit konzentrierter Natronlauge auf pH 2 bis 3 eingestellt. Der ausgefallene Feststoff wird abgesaugt, mit Wasser gewaschen und getrocknet.
Ausbeute: 15 g (64% d.Th.); Fp. 200°C (aus Ethanol).

*Beispiel 5*

5-Chlor-3-methyl-8-chinolincarbonsäure
(Verbindung Nr. 4)

Zu einer Lösung von 17 g 4-Chlor-anthranilsäure und 19 g Natrium-m-nitrobenzolsulfonat in 200 g 57%iger Schwefelsäure werden bei 100°C 14 g Methacrolein getropft. Nach 1stündigem Rühren bei 130°C wird mit 450 g Wasser verdünnt und heiss abgesaugt. Unter Kühlung wurde mit konzentrierter Natronlauge auf pH 2 bis 3 eingestellt. Der ausgefallene Feststoff wurde abgesaugt, mit Wasser gewaschen und getrocknet.
Ausbeute: 14 g (64% d.Th.); Fp. 165°C.

*Beispiel 6*

5,7-Dichlor-3-methyl-8-chinolincarbonsäure
(Verbindung Nr. 5)

Zu einer Lösung von 20,6 g 4,6-Dichloranthranilsäure und 19 g Natrium-m-nitrobenzolsulfonat in 200 g 57%iger Schwefelsäure werden bei 100°C 14 g Methacrolein getropft. Nach 1stündigem Rühren bei 130°C wird mit 450 g Wasser verdünnt und heiss abgesaugt. Unter Eiskühlung wurde mit konzentrierter Natronlauge auf pH 2 bis 3 eingestellt. Der ausgefallene Feststoff wird abgesaugt, mit Wasser gewaschen und getrocknet.
Ausbeute: 13 g (50% d.Th.); Fp. 220°C.

Analog können folgende Verbindungen der Formel I hergestellt werden:

| Verbindung Nr. | $X_n$ | Y | $R^1$ | $R^2$ | Schmp. [°C] |
|---|---|---|---|---|---|
| 6 | 7-Cl | O | $CH_3O$ | $CH_3$ | |
| 7 | 7-Cl | O | $C_2H_5O$ | $CH_3$ | |
| 8 | 7-Cl | O | n-$C_4H_9O$ | $CH_3$ | |
| 9 | 7-Cl | O | n-$C_5H_{11}O$ | $CH_3$ | |
| 10 | 7-Cl | O | n-$C_6H_{13}O$ | $CH_3$ | |
| 11 | 7-Cl | O | $OCH_3$ | $C_2H_5$ | |
| 12 | 7-Cl | O | $OC_2H_5$ | $C_2H_5$ | |
| 13 | 7-Cl | O | $O^{\ominus}Na^{\oplus}$ | $CH_3$ | |
| 14 | 7-Cl | O | $O^{\ominus}Na^{\oplus}$ | $C_2H_5$ | |
| 15 | 7-Cl | O | $O^{\ominus}\overset{\oplus}{N}H_3[(CH_2)_2OH]$ | $CH_3$ | |
| 16 | 7-Cl | O | $O^{\ominus}\overset{\oplus}{N}H_2[(CH_2)_2OH]_2$ | $CH_3$ | |
| 17 | 7-Cl | O | $O^{\ominus}\overset{\oplus}{N}H_3[(CH_2)_2OH]$ | $C_2H_5$ | |
| 18 | 7-Cl | O | $O^{\ominus}\overset{\oplus}{N}H_2[(CH_2)_2OH]_2$ | $C_2H_5$ | |
| 19 | 7-Cl | O | $NH_2$ | $CH_3$ | |
| 20 | 7-Cl | O | $N(C_2H_5)_2$ | $CH_3$ | |
| 21 | 7-Cl | O | 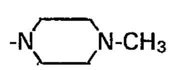 | $CH_3$ | |
| 22 | 7-Cl | O | $N(CH_2$-$CH_2OH)_2$ | $CH_3$ | |
| 23 | 7-Cl | O | 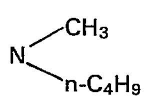 | $CH_3$ | |
| 24 | 7-Cl | O | OH | $CH_2Br$ | 220 |
| 25 | 7-Cl | O | $OCH_3$ | $CH_2Br$ | |
| 26 | 7-Cl | O | $OC_2H_5$ | $CH_2Br$ | |

*(Fortsetzung)*

| Verbindung Nr. | $X_n$ | Y | $R^1$ | $R^2$ | Schmp. [°C] |
|---|---|---|---|---|---|
| 27 | 5,7-$Cl_2$ | O | $OCH_3$ | $CH_3$ | |
| 28 | 5,7-$Cl_2$ | O | $OC_2H_5$ | $CH_3$ | |
| 29 | 5,7-$Cl_2$ | O | n-$C_4H_9O$ | $CH_3$ | |
| 30 | 5,7-$Cl_2$ | O | n-$C_5H_{11}O$ | $CH_3$ | |
| 31 | 5,7-$Cl_2$ | O | n-$C_6H_{13}O$ | $CH_3$ | |
| 32 | 5,7-$Cl_2$ | O | $CH_3O$ | $C_2H_5$ | |
| 33 | 5,7-$Cl_2$ | O | $C_2H_5O$ | $C_2H_5$ | |
| 34 | 5,7-$Cl_2$ | O | $O^{\ominus}Na^{\oplus}$ | $CH_3$ | |
| 35 | 5,7-$Cl_2$ | O | $O^{\ominus}Na^{\oplus}$ | $C_2H_5$ | |
| 36 | 5,7-$Cl_2$ | O | $O^{\ominus}\overset{\oplus}{N}H_3[(CH_2)_2OH]$ | $CH_3$ | |
| 37 | 5,7-$Cl_2$ | O | $O^{\ominus}\overset{\oplus}{N}H_2[(CH_2)_2OH]_2$ | $CH_3$ | |
| 38 | 5,7-$Cl_2$ | O | $O^{\ominus}\overset{\oplus}{N}H_3[(CH_2)_2OH]$ | $C_2H_5$ | |
| 39 | 5,7-$Cl_2$ | O | $O^{\ominus}\overset{\oplus}{N}H_2[(CH_2)_2OH]_2$ | $C_2H_5$ | |
| 40 | 5,7-$Cl_2$ | O | $NH_2$ | $CH_3$ | |
| 41 | 5,7-$Cl_2$ | O | $N(C_2H_5)_2$ | $CH_3$ | |
| 42 | 5,7-$Cl_2$ | O | 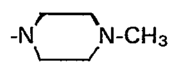 | $CH_3$ | |
| 43 | 5,7-$Cl_2$ | O | $N(CH_2$-$CH_2OH)_2$ | $CH_3$ | |
| 44 | 5,7-$Cl_2$ | O | 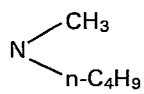 | $CH_3$ | |
| 45 | 5,7-$Cl_2$ | O | OH | $CH_2Br$ | |
| 46 | 5,7-$Cl_2$ | O | $OCH_3$ | $CH_2Br$ | |
| 47 | 5,7-$Cl_2$ | O | $OC_2H_5$ | $CH_2Br$ | |
| 48 | 7-Cl | O | OH | COOH | 260 |
| 49 | 7-Cl | O | OH | n-$C_3H_7$ | 200 |
| 50 | 7-Cl | O | OH | $CH_2P^{\oplus}(C_6H_5)_3Br^{\ominus}$ | 260 |
| 51 | 7-Cl | O | OH | H | 244 |

Die Verbindungen der Formel I können beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten zwischen 0,1 und 95 Gew.-%, vorzugsweise zwischen 0,5 und 90 Gew.-%, Wirkstoff.

Beispiele für Formulierungen sind:

I. Man vermischt 90 Gewichtsteile des Wirkstoffs Nr. 1 mit 10 Gewichtsteilen N-Methyl-α-pyrrolidon und erhält eine Lösung, die zur Anwendung in Form kleinster Tropfen geeignet ist.

II. 20 Gewichtsteile des Wirkstoffs Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N--mono-ethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100000 Gewichtsteilen Wasser erhält man eine wässrige Dispersion, die 0,02 Gewichtsprozent des Wirkstoffs enthält.

III. 20 Gewichtsteile des Wirkstoffs Nr. 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingiessen und feines Verteilen der Lösung in 100000 Gewichtsteilen Wasser erhält man eine wässrige Dispersion, die 0,02 Gewichtsprozent des Wirkstoffs enthält.

IV. 20 Gewichtsteile des Wirkstoffs Nr. 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in 100000 Gewichtsteilen Wasser erhält man eine wässrige Dispersion, die 0,02 Gewichtsprozent des Wirkstoffs enthält.

V. 20 Gewichtsteile des Wirkstoffs Nr. 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gewichtsprozent des Wirkstoffs enthält.

VI. 3 Gewichtsteile des Wirkstoffs Nr. 24 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 49 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 5 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls vermischt. Man erhält eine stabile ölige Dispersion.

Die Applikation der Wirkstoffe bzw. der Mittel kann im Vorauflaufverfahren oder bei Nachauflaufanwendung erfolgen. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,05 bis 5 kg/ha, vorzugsweise 0,5 bis 3 kg/ha.

Die herbizide Wirkung von Verbindungen der Formel I wird durch Gewächshausversuche gezeigt:

Als Kulturgefässe dienen Plastikblumentöpfe mit 300 $cm^3$ Inhalt und lehmigem Sand mit etwa 1,5% Humus als Substrat. Bei Reis werden grössere Anteile Torf zugesetzt, um ein besseres Wachstum zu gewährleisten. Die Samen der Testpflanzen werden nach Arten getrennt flach eingesät. Unmittelbar danach werden bei Vorauflaufanwendung die Wirkstoffe auf die Erdoberfläche aufgebracht. Sie werden hierzu in Wasser als Verteilungsmittel suspendiert oder emulgiert und mittels fein verteilender Düsen gespritzt. Die Aufwandmengen betragen dabei beispielsweise 0,5 und 2,0 kg Wirkstoff/ha.

Nach dem Aufbringen der Wirkstoffe werden die Gefässe leicht beregnet, um Keimung und Wachstum in Gang zu bringen. Danach werden die Gefässe mit durchsichtigen Plastikhauben abgedeckt, bis die Pflanzen angewachsen sind. Diese Abdeckung bewirkt ein gleichmässiges Keimen der Testpflanzen, sofern diese nicht durch die Wirkstoffe beeinträchtigt werden.

Für die Nachauflaufbehandlung werden die Testpflanzen je nach Wuchsform erst bis zu einer Wuchshöhe von 3 bis 15 cm angezogen und danach behandelt. Zur Nachauflaufbehandlung werden entweder direkt gesäte und in den gleichen Gefässen aufgewachsene Pflanzen ausgewählt, oder aber sie werden erst als Keimpflanzen in Saatschalen getrennt angezogen und einige Tage vor der Behandlung in die Versuchsgefässe verpflanzt. Eine Abdeckung unterbleibt bei der Nachauflaufbehandlung. Die Aufwandmengen für die Nachauflaufbehandlung betragen beispielsweise 0,5, 1,0 und 3,0 kg Wirkstoff/ha.

Die Versuchsgefässe werden im Gewächshaus aufgestellt, wobei für wärmeliebende Arten wärmere Bereiche (20 bis 35°C) und für solche gemässigter Klimate 10 bis 20°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Apium graveolens (Sellerie), Avena sativa (Hafer), Brassica napus (Raps), Beta vulgaris (Zuckerrübe), Cassia tora, Centaurea cyanus (Kornblume), Daucus carota (Möhre), Galium aparine (Klettenlabkraut), Hordeum vulgare (Gerste), Ipomoea Spp. (Prunkwindearten), Lamium amplexicaule (Stengelumfassende Taubnessel), Mentha piperita (Pfefferminz), Oryza sativa (Reis), Secale cereale (Roggen), Solanum nigrum (Schwarzer Nachtschatten), Triticum aestivum (Weizen), Veronica spp. (Ehrenpreisarten), Veronica persica (Persischer Ehrenpreis), Zea Mays (Mais).

Als Vergleichsmittel werden folgende substituierte Chinolinderivate eingesetzt:

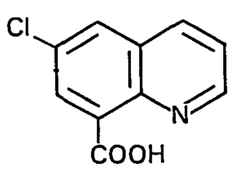

(A; US-PS 4 036 963),

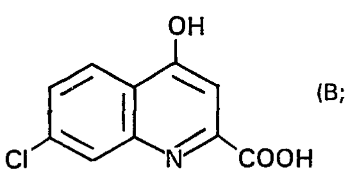

(B; US-PS 2 661 276),

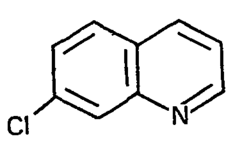

(C; DE-OS 23 22 143).

Die Aufwandmengen entsprechen denen der erfindungsgemässen Verbindungen in den jeweiligen Versuchen.

Vorauflaufanwendung:

Verbindung Nr. 2 zeigt beispielsweise mit 0,5 kg Wirkstoff/ha eine gute herbizide Aktivität gegen typische Unkräuter oder auch gegen Kulturpflanzenarten als Vertreter der Pflanzenfamilie der Umbelliferen. Gleichzeitig ist die Verbindung gut verträglich für eine Reihe landwirtschaftlicher Kulturpflanzen. Dasselbe gilt für Verbindung Nr. 3, die mit 2,0 kg Wirkstoff/ha für Kulturpflanzen selektiv ebenfalls unerwünschte Pflanzen bekämpft.

Nachauflaufanwendung:

Bei der Prüfung auf herbizide Wirkung bekämpft beispielsweise die Verbindung Nr. 2 mit 0,5, 1,0 und 3,0 kg/ha unerwünschte Pflanzen gut, ohne Kulturpflanzen, wie Hafer, Raps oder Gerste, zu schädigen. Die Verbindung Nr. 24 ist mit 0,5 kg/ha bei gleichmässiger Selektivität in Zuckerrüben, Mais und Weizen besonders wirksam gegen Galium aparine. Verbindung Nr. 3 ist gegen breitblättrige Unkräuter gut wirksam, wobei 0,5 kg Wirkstoff/ha für Raps und Gerste verträglich sind, während von Kulturhafer 3,0 kg Wirkstoff/ha toleriert werden. Weiterhin zeigen beispielsweise die Verbindungen Nr. 4 und 5 mit 3,0 kg/ha eine beachtliche herbizide Wirkung.

Die als Vergleichsmittel herangezogenen Verbindungen A, B und C sind den Chinolinderivaten der Formel I in ihrer herbiziden Wirkung weit unterlegen.

In Anbetracht der guten Verträglichkeit für zahlreiche breitblättrige und andere Kulturen und der Vielseitigkeit der Applikationsmethoden können die erfindungsgemässen Wirkstoffe oder diese enthaltende Mittel noch in einer weiteren grossen Zahl von Kulturpflanzen zur Beseitigung unerwünschten Pflanzenwuchses eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Asparagus officinalis | Spargel |
| Avena sativa | Hafer |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica napus var. rapa | Weisse Rübe |
| Brassica rapa var. silvestris | Rübsen |
| Camellia sinensis | Teestrauch |
| Carthamus tinctorius | Saflor - Färberdistel |
| Carya illinoinensis | Pekannussbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |

*(Fortsetzung)*

| Botanischer Name | Deutscher Name |
|---|---|
| Cynodon dactylon | Bermudagras |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süsskartoffeln |
| Juglans regia | Walnussbaum |
| Linum usitatissimum | Faserlein |
| Lycopersicon lycopersicum | Tomate |
| Malus spp. | Apfel |
| Manihot esculenta | Maniok |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Pennisetum glaucum | Perl- oder Rohrkolbenhirse |
| Picea abies | Rotfichte |
| Abies alba | Weisstanne |
| Pinus spp. | Kiefer |
| Pisum sativum | Gartenerbse |
| Prunus avium | Süsskirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preisselbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemässen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate und andere in Betracht.

Geeignete Mischungspartner sind insbesondere Harnstoffderivate, wie 3-(3-Chlor-4-methyl-phenyl)-1,1-dimethyl-harnstoff oder 3-(4-Isopropyl-phenyl)-1,1-dimethyl-harnstoff. Diese Harnstoffe werden vorzugsweise mit Chinolinderivaten der Formel I kombiniert, bei denen X Chlor in 7-Stellung, n 1, $R^1$ Wasserstoff oder die Gruppe OM, wobei M Wasserstoff, ein Äquivalent Alkalimetallion oder ein Dialkylammoniumion mit 1 bis 4 C-Atomen in einer Alkylgruppe bedeutet, $R^2$ $C_1$-$C_4$-Alkyl und Y Sauerstoff bedeuten.

Eine sehr gute herbizide Wirkung haben beispielsweise Mischungen, die 3-Methyl-7-chlor-8-carboxy-chinolin oder 3-Ethyl-7-chlor-8-carboxy-chinolin und einen der beiden obengenannten Harnstoffe enthalten.

Weiterhin kommen folgende Wirkstoffe als Mischungspartner in Betracht:

3-(1-Methylethyl)-1H-2,1,3-benzothiadiazin-4(3H)-on-2,2-dioxid und Salze

3-(1-Methylethyl)-8-chlor-1H-2,1,3-benzothiadiazin-4(3H)-on-2,2-dioxid und Salze

3-(1-Methylethyl)-8-fluor-1H-2,1,3-benzothiadiazin-4(3H)-on-2,2-dioxid und Salze

3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid

1-Methoxymethyl-8-chlor-3-(1-methylethyl)-2,1,3-benzothiadiazin-4(3H)-on-2,2-dioxid

1-Methoxymethyl-8-fluor-3-(1-methylethyl)-2,1,3-benzothiadiazin-4(3H)-on-2,2-dioxid

1-Cyan-8-chlor-3-(1-methylethyl)-2,1,3-benzothiadiazin-4(3H)-on-2,2-dioxid

1-Cyan-8-fluor-3-(1-methylethyl)-2,1,3-benzothiadiazin-4(3H)-on-2,2-dioxid

1-Cyan-8-methyl-3-(1-methylethyl)-2,1,3-benzothiadiazin-4(3H)-on-2,2-dioxid

1-Azidomethyl-3-(1-methylethyl)-2,1,3-benzothiadiazin-4(3H)-on-2,2-dioxid

3-(1-Methylethyl)-1H-(pyridino-[3,2-e]2,1,3-thiadiazin-(4)-on-2,2-dioxid

1-Cyan-3-(1-methylethyl)-2,1,3-benzothiadiazin-4(3H)-on-2,2-dioxid

N-(1-Ethylpropyl)-2,6-dinitro-3,4-dimethylanilin

N-(1-Methylethyl)-N-ethyl-2,6-dinitro-4-trifluormethyl-anilin

N-n-Propyl-N-β-chlorethyl-2,6-dinitro-4-trifluormethyl-anilin

N-n-Propyl-N-cyclopropylmethyl-2,6-dinitro-4-trifluor-methyl-anilin

N,N-Di-n-propyl-2,6-dinitro-3-amino-4-trifluormethylanilin

N,N-Di-n-propyl-2,6-dinitro-4-methyl-anilin
N,N-Di-n-propyl-2,6-dinitro-4-methylsulfonyl-anilin
N,N-Di-n-propyl-2,6-dinitro-4-aminosulfonyl-anilin
N,N-Di-beta-chlorethyl-2,6-dinitro-4-methyl-anilin
N-Ethyl-N-(2-methylallyl)-2,6-dinitro-4-trifluormethyl-anilin
N-Methylcarbaminsäure-3,4-dichlorbenzylester
N-Methylcarbaminsäure-2,6-di-tert.butyl-4-methylphenyl-ester
N-Phenylcarbaminsäure-isopropylester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionanilid
Ethyl-N-[3-(N'-phenylcarbamoyloxy)-phenyl]-carbamat
Methyl-N-[3-(N'-methyl-N'-phenylcarbamoyloxy)-phenyl]-carbamat
Isopropyl-N-[3-(N'-ethyl-N'-phenylcarbamoyloxy)-phenyl]-carbamat
Methyl-N-[3-(N'-methylphenylcarbamoyloxy)-phenyl]-carbamat
Ethyl-N-[3-(N'-3-chlor-4-fluorphenylcarbamoyloxy)-phenyl]-carbamat
Ethyl-N-[3-(N'-3,4-difluorphenylcarbamoyloxy)-phenyl]-carbamat
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäure-ethylester
S-Ethyl-hexahydro-1H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
Alpha,alpha-Dichlorpropionsäure-Natriumsalz
Alpha,alpha-Dichlorbuttersäure-Natriumsalz
Alpha,alpha,beta,beta-Tetrafluorpropionsäure-Natriumsalz
Alpha-Methyl-alpha,beta-dichlorpropionsäure-Natriumsalz
Alpha-Chlor-beta-(4-chlorphenyl)-propionsäure-methylester
Alpha,beta-Dichlor-beta-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäureNatriumsalz
2-[4-(3',5'-Dichlorpyridyl-2-oxy)-phenoxy]-propionsäureNatriumsalz
2-(N-Benzoyl-N-(3,4-dichlorphenyl)-amino)-propionsäureethylester
2-(N-Benzoyl-3-chlor-4-fluorphenylamino)-propionsäure-methylester
2-(N-Benzoyl-3-chlor-4-fluorphenylamino)-propionsäureisopropylester
2-Chlor-4-ethylamino-6-isopropylamino-1,3,5-triazin
2-Chlor-4-ethylamino-6-(amino-2'-propionitril)-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin
2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion
3-tert.Butyl-5-chlor-6-methyluracil
3-Isopropyl-5-brom-6-methyluracil

3-sec.Butyl-5-brom-6-methyluracil
3-Cyclohexyl-5,6-trimethylenuracil
2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-(4-Chlorphenoxy)-3,3-dimethyl-1-(H-1,2,4-triazolyl)-2-butanon
N,N-Diallylchloracetamid
N-Isopropyl-2-chloracetanilid
N-(1-Methyl-propin-2-yl)-2-chloracetanilid
2-Methyl-6-ethyl-N-ethoxymethyl-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracet-anilid
2-Methyl-6-ethyl-N-(pyrazolyl-methyl)-2-chloracetanilid
2,6-Dimethyl-N-(pyrazolyl-methyl)-2-chloracetanilid
2,6-Dimethyl-N-(4-methylpyrazolyl-methyl)-2-chloracetanilid
2,6-Dimethyl-N-(1,2,4-triazolyl-methyl)-2-chloracetanilid
2,6-Dimethyl-N-(3,5-dimethylpyrazolyl-methyl)-2-chloracetanilid
2,6-Dimethyl-N-(1,3-dioxalan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2-chloracetanilid
2,6-Diethyl-N-(n-butoxymethyl)-2-chloracetanilid
2,6-Diethyl-N-ethoxycarbonylmethyl-2-chloracetanilid
2,3-Dimethyl-N-isopropyl-2-chloracetanilid
2,6-Diethyl-N-(2-n-propoxy-ethyl)-2-chloracetanilid
alpha-(2-Methyl-4-chlorphenoxy)-N-methoxy-acetamid
2-(alpha-Naphthoxy)-N,N-diethylpropionamid
2,2-Diphenyl-N,N-dimethylacetamid
alpha-(3,4,5-Tribrompyrazolyl)-N,N-dimethylpropionamid
N-(1,1-Dimethylprop-2-inyl)-3,5-dichlorbenzamid
N-Naphth-1-yl-phthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid
2-Propionyl-amino-4-methyl-5-chlor-thiazol
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-O-2,4-dinitrophenylbenzaldoxim (Salze)
Pentachlorphenyl-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-Isopropylcarbamoyl-oxyphenyl)-4-methyl-1,2,4-oxadiazolidin-3,5-dion
2-Phenyl-3,1-benzoxazinon-(4)
3-(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methan-sulfonat
2-Methyl-4,6-dinitrophenol (Salze, Ester)
3-(4-Chlorphenyl)-3,4,5,9,10-pentaazatetracyclo-[5,4,1,$0^{2,6}$,$0^{8,1}$]-dodeca-3,9-dien
2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat
2-sec.Amyl-4,6-dinitrophenol (Salze, Ester)
1-(alpha,alpha-Dimethylbenzyl)-3-(4-methylphenyl)-harnstoff
1-Phenyl-3-(2-methylcyclohexyl)-harnstoff
1-(4-Chlorphenyl)-3,3-dimethyl-harnstoff
1-(4-Chlorphenyl)-3-methyl-3-(butin-1-yl-3)-harnstoff
1-(3,4-Dichlorphenyl)-3,3-dimethyl-harnstoff
1-(3,4-Dichlorphenyl)-3-methyl-3-n-butyl-harnstoff
1-(3-Trifluormethylphenyl)-3,3-dimethyl-harnstoff
1-(alpha,alpha,beta,beta-Tetrafluorethoxyphenyl)-3,3-dimethyl-harnstoff
1-(3-tert.Butylcarbamoyloxy-phenyl)-3,3-dimethyl-harnstoff
1-(3-Chlor-4-methoxyphenyl)-3,3-dimethyl-harnstoff
1-[4(4'-Chlorphenoxy)-phenyl]-3,3-dimethyl-harnstoff
1-[4(4'-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-isobutylamid
1,2-Dimethyl-3,5-diphenylpyrazolium-methylsulfat

1,3-Dimethyl-4-(3,4-dichlorbenzoyl)-5-(4-methylphenylsulfonyloxy)-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
1,1'-Dimethyl-4,4'-dipyridylium-dimethylsulfat
1,1'-Di(3,5-dimethylmorpholin-carbonylmethyl)-4,4'-dipyridylium-dichlorid
1,1'-Ethylen-2,2'-dipyridylium-dibromid
3-[1-(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino)-butyliden]-5,5-dimethylcyclohexan-1,3-dion (Salze)
2-[1-(N-Allyloxyamino)-butyliden]-5,5-dimethyl-4-methoxycarbonyl-cyclohexan-1,3-dion (Salze)
2,4-Dichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
Alpha-Naphthoxyessigsäuremethylester
2-(2,4-Dichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chlorethanphosphonsäure-2-chlorethylester
Ammonium-ethyl-carbamoyl-phosphonat
O,O-Di-n-butyl-(1-n-butylamino-cyclohexyl)-phosphonat
Trithiobutylphosphit
O,O-Diisopropyl-5-(2-benzolsulfonylamino-ethyl)-phosphordithioat
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N,N-dimethylhydrazid (Salze)
(2-Chlorethyl)-trimethyl-ammoniumchlorid
(2-Methyl-4-phenylsulfonyl)-trifluormethansulfonanilid
Ammoniumrhodanid
Calciumcyanamid
2-Chlor-4-trifluormethylphenyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Iodpyridyl-2-oxy)-phenoxy]-propionsäure-n-butylester
2-Chlor-4-trifluormethylphenyl-3'-(2-fluorethoxy)-4'-nitro-phenylether
2-Chlor-4-trifluormethylphenyl-3'-ethoxycarbonylmethylthio-4'-nitrophenylether
2,4,6-Trichlorphenyl-3'-ethoxycarbonyl-methylthio-4'-nitrophenylether
2-[1-(N-ethoxyamino)-butyliden]-5-(2-ethylthiopropyl)-3-hydroxy-cyclohexen-(2)-on-(1) (Salze)
2-[1-(N-ethoxyamino)-butyliden]-5-(2-phenylthiopropyl)-3-hydroxy-cyclohexen-(2)-on-(1) (Salze)
4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-alpha,alpha,beta-trifluor-beta-bromethoxyphenyl)-3-(2H)-pyridazinon
2,4-Dichlor-3'[2-(2-ethoxy-ethoxy)-ethoxy]-4'-nitro-diphenyl-ether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1-(3-Chlor-4-ethoxyphenyl)-3,3-dimethylharnstoff
2-Methyl-4-chlorphenoxy-thioessigsäureethylester
2-Chlor-3,5-dijod-4-acetoxy-pyridin
1-(4-[2-(4-Methylphenyl)-ethoxy]-phenyl)-3-methyl-3-methoxyharnstoff
2,6-Dimethyl-N-(pyrazolyl-methylenoxymethyl)-2-chloracetanilid
2-Methyl-6-ethyl-N-(pyrazolyl-methylenoxymethyl)-2-chloracetanilid
alpha-2,4-(Dichlorphenoxy-propionsäure)-(3-methoxycarbonylamino)-anilid
1-(alpha-2-Brom-4-chlorphenoxypropionsäure)-3-(O-methylcarbamoyl)-anilid
2-Methyl-6-ethyl-N-(pyrazolyl-ethylenoxymethyl)-2-chloracetanilid
2-(3-Trifluormethylphenyl)-4H-3,1-benzoxazin-4-on
2-(3-Pentafluorethoxyphenyl)-4H-3,1-benzoxazin-4-on
2-(3-Trifluormethylthio-phenyl)-4H-3,1-benzoxazin-4-on
2-(3-Difluorchlormethoxyphenyl)-4H-3,1-benzoxazin-4-on
5-Nitro-2-(3-trifluormethyl-phenyl)-4H-3,1-benzoxazin-4-on
5-Chlor-2-(3-trifluormethoxyphenyl)-4H-3,1-benzoxazin-4-on
5-Chlor-2-(3-alpha,alpha,beta,beta-tetrafluorethoxyphenyl)-4H-3,1-benzoxazin-4-on
5-Fluor-2-(3-alpha,alpha,beta,beta-tetrafluorethoxyphenyl)-4H-3,1-benzoxazin-4-on
5-Chlor-2-(4-difluorchlormethoxyphenyl)-4H-3,1-benzoxazin-4-on

5-Fluor-2-(4-difluorchlormethoxyphenyl)-4H-3,1-benzoxazin-4-on
5-Fluor-2-phenyl-4H-3,1-benzoxazin-4-on
5-Fluor-2-(3-difluormethoxyphenyl)-4H-3,1-benzoxazin-4-on
5-Chlor-2-phenyl-4H-3,1-benzoxazin-4-on
N-3-Chlor-4-isopropylphenyl-thiolcarbaminsäuremethylester
6-Methyl-3-methoxy-5,6-dihydro-1,2,4,6-thiatriazin-5-on-1,1-dioxid
Natriumsalz
6-Methyl-3-ethoxy-5,6-dihydro-1,2,4,6-thiatriazin-5-on-1,1-dioxid
5-Amino-4-chlor-2-phenyl-3(2H)-pyridazinon
5-Amino-4-brom-2-phenyl-3(2H)-pyridazinon
5-Methylamino-4-chlor-2-(3-trifluormethylphenyl)-3(2H)-pyridazinon
5-Methylamino-4-chlor-2-(3-alpha,alpha,beta,beta-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
1-[3'-(2''-Chlor-4''-trifluormethylphenoxy)]-phenyl-4,5-dimethoxy-pyridazinon-6
1-[4'-(3''-Trifluormethyl-phenoxy)]-phenyl-4,5-dimethoxy-pyridazinon-6
N-[4-(4'-Methoxy-phenoxy)-3-chlor-phenyl]-carbaminsäuremethylester
N-[4-(4'-Difluormethoxy-phenoxy)-3-chlor-phenyl]-thio-carbaminsäuremethylester
N-[4-(4'-Difluormethoxy-phenoxy)-phenyl]thio-carbaminsäuremethylester
1-[4-(4'-Methylphenylpropyl)-phenyl]-3-methyl-3-methoxyharnstoff
1-[3-(4'-Chlorphenyl-propyl)-phenyl]-3-methyl-3-methoxyharnstoff
1-[4-(3-Phenyl-2-methyl-propyl)-phenyl]-3-methyl-3-methoxyharnstoff
1-[4-(3-(4'-Chlorphenyl)-2-methyl-propyl)-phenyl]-3-methyl-3-methoxyharnstoff
1-[4-(3-(4'-Methylphenyl)-2-methylpropyl)-phenyl]-3-methyl-3-methoxyharnstoff
2-[1-(N-Ethyloxyamino)-butyliden]-5(4-ethylphenyl)-3-hydroxy-cyclohexen-(2)-on-(1) (Salze)
2-[1-(N-Ethyloxyamino)-butyliden]-5(4-fluorphenyl)-3-hydroxy-cyclohexen-(2)-on-(1) (Salze)
2-[1-(N-Ethyloxyamino)-butyliden]-5-(4-chlorphenyl)-3-hydroxy-cyclohexen-(2)-on-(1) (Salze)
2'-(2,4,6-Trichlorphenyl)-hydrazino-2-cyanacrylsäuremethylester
2-[1-(N-Ethyloxyamino)-butyliden]-5-(1,3,3-trimethyl-cyclohexen-1-yl-2)-3-hydroxy-cyclohexen-(2)-on-(1) (Salze)
2-[1-(N-Ethyloxyamino)-butyliden]-5-(2,4,4-trimethyl-cyclohexen-1-yl-3)-3-hydroxy-cyclohexen-(2)-on-(1) (Salze)
2-[1-(N-3-Chlorallyl-oxamino)-butyliden]-5-(1-methyl-cyclohex-1-en-4-yl)-3-hydroxy-cyclohexen-(2)-on-(1) (Salze)
3-Isobutoxy-5-methyl-4-methoxycarbonyl-pyrazol
5-Amino-1-(2,4,6-trichlorphenyl)-4-cyano-pyrazol
5-Amino-1-(2,4,6-tribromphenyl)-4-cyano-pyrazol
5-Amino-1-(2,4,6-trichlorphenyl)-4-methoxycarbonyl-pyrazol
5-Amino-(2,4-dichlor-6-bromphenyl)-4-methoxycarbonyl-pyrazol
5-Amino-(2,6-dichlor-4-bromphenyl)-4-methoxycarbonyl-pyrazol
5-Chlor-2-(3-trifluormethyl-phenyl)-4H-3,1-benzoxazin-4-on
5-Fluor-2-(3-trifluormethyl-phenyl)-4H-3,1-benzoxazin-4-on
2-(3-Tetrafluorethoxyphenyl)-4H-3,1-benzoxazin-4-on
5-Chlor-2-(4'-fluorphenyl)-4H-3,1-benzoxazin-4-on
5-Fluor-2-(4'-fluorphenyl)-4H-3,1-benzoxazin-4-on
5-Fluor-2-(3'fluorphenyl)-4H-3,1-benzoxazin-4-on
5-Chlor-2-(3'-fluorphenyl)-4H-3,1-benzoxazin-4-on
5-Chlor-2-(3'-difluorchlormethylphenyl)-4H-3,1-benzoxazin-4-on
5-Fluor-2-(3'-difluorchlormethylphenyl)-4H-3,1-benzoxazin-4-on
6-Methyl-3-methoxy-5-(4'-nitrophenoxy)-6H-1,2,4,6-thiatriazin-1,1-dioxid
6-Methyl-3-methoxy-5-(propargyloxy)-6H-1,2,4,6-thiatriazin-1,1-dioxid
6-Methyl-3-methoxy-5-(2,4-dichlorbenzoxy)-6H-1,2,4,6-thiatriazin-1,1-dioxid
2-(2',4'-Dichlorphenoxy)-2-fluorpropionsäure (Salze, Ester)
2-[4-(5'-Trifluormethylpyridyl-2-oxy)-phenoxy]-propionsäurebutylester
2-[4-(3'-Chlor-5'-trifluormethylpyridyl-2-oxy)-phenoxy]-propionsäure (Salze, Ester)
2-[4-(6-Chlorchinoxalyl-2-oxy)-phenoxy]-propionsäure-pentylester
2-[4-(6-Chlor-chinoxalyl-2-oxy)-phenoxy]-propionsäuremethylester
2-[4-(6-Chlorbenzthiazolyl-2-oxy)-phenoxy]-propionsäure (Salze, Ester)
2-[4-(6-Chlorbenzoxazolyl-2-oxy)-phenoxy]-propionsäure (Salze, Ester)
1-[5-(3-Fluorbenzylthio)-thiadiazolyl-2]-1-methylharnstoff
2-Methoxycarbonyl-N-(3,5-dimethylpyrimidinyl-2-aminocarbonyl)-benzolsulfonamid
alpha-(3,5,6-Trichlor-pyrid-2-yl-oxy)-essigsäure (Salze, Ester)
alpha-(4-Amino-3,5-dichlor-6-fluor-pyrid-2-yl-oxy)-essigsäure (Salze, Ester)
S-[N-(4-Chlorphenyl)-N-isopropyl-carbamoyl-methyl]-O,O-dimethyl-dithiophosphat
Ammonium-(3-amino-3-carboxy-propyl)-methylphosphinat
(Hydroxy)-(methyl)-phosphinyl-L-alpha-aminobutyryl-L-alanyl-Natriumsalz
4-Trifluormethyl-diphenylether
2-(3,5-Dichlorphenyl)-2-(2'2'2'-trichlorethyl)-oxiran
2,4-Diamino-5-methylthio-6-chlor-pyrimidin
N-(4-Ethylthio-2-trifluormethyl-phenyl)-methylsulfonamid
3-Methoxy-4-methyl-5(3-methyl-2-butenyloxy)-1,2-di(hydroxymethyl)-benzol
2-(3,5-Dimethylphenoxy)-2-(1,2,4-triazolyl-1)-essigsäure-N-tertiär-butylamid
2-(3,5-Dichlorphenoxy)-2-(1,2,4-triazolyl-1)-essigsäure-N-tertiär-butylamid
3,7-Dichlor-8-chinolincarbonsäure (Salze, Ester)
5-(2-Chlor-4-trifluormethyl-phenoxy)-N-(1-methoxycarbonylethoxy)-benzamid

N-[3-(1-Ethyl-1-methylpropyl)-isoxazolyl-5]-2,6-dimethoxybenzamid
2'-Methoxyethyl-2-[5-(2-chlor-4-trifluormethyl-phenoxy)-2-nitrophenoxy]-propionat
Methyl-6-(4-isopropyl-4-methyl-5-oxo-2-imidazolin-2-yl)-3-methylbenzoat
Methyl-6-(4-isopropyl-4-methyl-5-oxo-2-imidozin-2-yl)-4-methylbenzoat
Benzyltrimethylammoniumchlorid 1-[alpha-(4-Trifluormethyl-phenoxy)-phenoxy-propionsäure]-3-(O-methylcarbamoyl)-anilid
1-Dodecyl-cycloheptan-2-on
N-[2-Chlor-4-methylsulfonyl-phenyl]-chlormethansulfonamid
N-[2-Brom-4-ethylsulfonyl-phenyl]-chlormethansulfonamid
N-[2,3-Dichlor-4-(ethylsulfonyl)-phenyl]-chlormethansulfonamid
2-[1-(N-Ethoxyamino)-pyropyliden-]-5-(pyrid-3-yl)-3-hydroxy-cyclohex-2-en-1-on (Salze)
2,4,5-Trichlorphenoxyessigsäure (Salze, Ester, Amide)
2-[1-(N-Ethoxyamino)-butyliden]-5-(tetrahydropyran-3-yl)-3-hydroxy-cyclohex-2-en-1-on (Salze)
2-[1-(N-Ethoxyamino)-butyliden]-5-(4-methyl-tetrahydropyran-3-yl)-3-hydroxy-cyclohex-2-en-1-on (Salze)
2-[1-(N-Ethoxyamino)-butyliden]-5-(tetrahydrothiopyran-3-yl)-3-hydroxy-cyclohex-2-en-1-on (Salze)
2-[1-(N-Ethoxyamino)-propyliden]-5-(pyrid-3-yl)-3-hydroxy-cyclohex-2-en-1-on (Salze)
2-[1-(N-Allyloxamino)-propyliden]-5-(pyrid-3-yl)-3-hydroxy-cyclohex-2-en-1-on
2-[1-(N-Ethoxyamino)-butyliden]-5-(pyrid-3-yl)-3-hydroxy-cyclohex-2-en-1-on (Salze)
2-[1-(N-Allyloxyamino)-butyliden]-5-(pyrid-3-yl)-3-hydroxy-cyclohex-2-en-1-on (Salze)
2-[4,5-Dihydro-4-methyl-4-isopropyl-5-oxo-1H-imidazol-2-yl]-3-chinolincarbonsäure
2-[4,5-Dihydro-4-methyl-4-isopropyl-5-oxo-1H-imidazol-2-yl]-nicotinsäureisopropylaminsalz
2-Chlor-2'-methyl-6'-ethyl-N-(N'-1-methoxycarbonyl)-ureidomethylacetanilid
2-Chlor-2'-6'-diethyl-N-(N'-1-methoxycarbonyl)-ureidomethylacetanilid
2-Chlor-2'-6'-dimethyl-N-(N'-1-methoxycarbonyl)-ureidomethylacetanilid
2-Chlor-6-nitro-3-phenoxy-anilin
N-Phosphonomethyl-glycin-trimethyl-sulfoniumsalz
5-(2-Chlor-4-trifluormethyl-phenoxy)-2-nitro-N-methansulfonyl-benzamid
5-(3-Chlor-4-trifluormethyl-phenoxy)-2-nitro-benzoesäure-1-(ethoxycarbonyl-ethyl)-ester.

Ausserdem kann es von Nutzen sein, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

1. Chinolinderivate der Formel

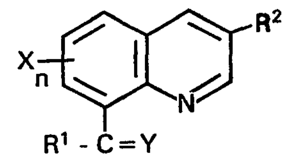

(I),

in der

X für Chlor in den Positionen 5 oder 7 steht, wobei n 1 oder 2 bedeutet,

Y für Sauerstoff oder Schwefel,

$R^1$ für Halogen, die Gruppe $-NR^3R^4$, worin $R^3$ und $R^4$ gleich oder verschieden sind und Wasserstoff, $C_{1-6}$-Alkyl, $C_{2-6}$-Alkenyl, $C_{1-6}$-Hydroxyalkyl, Cyclohexyl oder Phenyl oder $R^3$ und $R^4$ zusammen einen Tetramethylen- oder Pentamethylenrest, wobei eine $CH_2$-Gruppe durch ein Sauerstoff- oder Stickstoffatom oder die $N(CH_3)$-Gruppe ersetzt sein kann, bedeuten oder für die Gruppe OM steht, worin M ein Äquivalent eines Alkalimetall- oder Erdalkalimetallions, Wasserstoff, $C_{1-8}$-Alkyl oder den Rest $H_2N^{\oplus}R^3R^4$, in dem $R^3$ und $R^4$ die oben angegebenen Bedeutungen haben, bedeutet, und

$R^2$ für gegebenenfalls durch Halogen, Amino, Monoalkylamino, Dialkylamino oder Trialkylammonium mit 1 bis 4 Kohlenstoffatomen in einer Alkylgruppe, $C_{1-4}$-Alkoxy, $C_{1-4}$-Alkylthio, Trialkylphosphonium mit 1 bis 4 Kohlenstoffatomen in einer Alkylgruppe oder Triphenylphosphonium in ω-Stellung substituiertes $C_{1-6}$-Alkyl, für Formyl, Cyano, Carboxyl, Carbamoyl, N-Alkyl-carbamoyl oder N,N-Dialkylcarbamoyl mit 1 bis 4 Kohlenstoffatomen in einer Alkylgruppe oder für $C_{2-6}$-Alkenyl stehen oder in der der Rest $-\underset{\underset{Y}{\|}}{C}-R^1$ eine Nitrilgruppe oder eine Halogenmethylgruppe ist.

2. Chinolinderivate der Formel I gemäss Anspruch 1, dadurch gekennzeichnet, dass X Chlor in 7-Stellung, n 1, $R^1$ die Gruppe OM, wobei M Wasserstoff, ein Äquivalent Alkalimetallion oder ein Dialkylammoniumion mit 1 bis 4 C-Atomen in einer Alkylgruppe bedeutet, $R^2$ $C_{1-4}$-Alkyl und Y Sauerstoff bedeuten.

3. Verfahren zur Herstellung von Chinolinderivaten der Formel I gemäss Anspruch 1, dadurch gekennzeichnet, dass man eine Verbindung der Formel

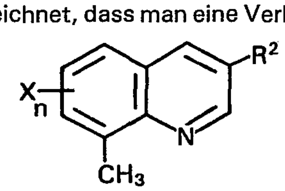

(II),

in der X, n und $R^2$ die im Anspruch 1 genannten Bedeutungen haben, mit einem Halogenierungsmittel in Gegenwart eines Radikalstarters bei einer Temperatur zwischen 40 und 140 °C halogeniert und die so erhaltenen Verbindungen der Formel

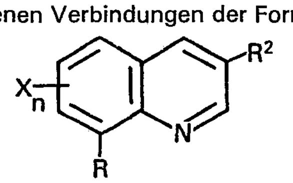

(III),

in der X, n und $R^2$ die im Anspruch 1 genannten Bedeutungen haben und R Halogenmethyl bedeutet, gegebenenfalls in die Verbindung der Formel I überführt, bei denen der Rest - C - $R^1$ nicht für eine Ha-
||
Y
logenmethylgruppe steht.

4. Verfahren zur Herstellung von Chinolinderivaten der Formel I, in der $R^1$ für die Gruppe OM, wobei M Wasserstoff bedeutet, und Y für Sauerstoff stehen und X, n und $R^2$ die im Anspruch 1 genannten Bedeutungen haben, dadurch gekennzeichnet, dass man eine Verbindung der Formel

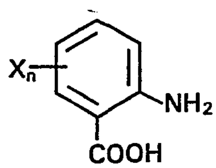
(IV),

in der X und n die im Anspruch 1 genannten Bedeutungen haben, mit einem Aldehyd der Formel

$H_2C=C - CHO$ (V),
|
$R^2$

in der $R^2$ die im Anspruch 1 genannten Bedeutungen hat, umsetzt.

5. Verfahren zur Herstellung von Chinolinderivaten der Formel I, in der X, n, $R^1$ und Y die im Anspruch 1 genannten Bedeutungen haben und $R^2$ für Brommethyl oder Dibrommethyl steht, dadurch gekennzeichnet, dass man eine Verbindung der Formel

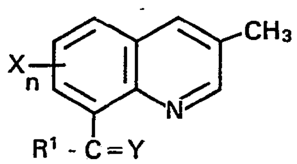
(VI),

in der X, n, $R^1$ und Y die im Anspruch 1 genannten Bedeutungen haben, mit einem Brom liefernden Agens umsetzt.

6. Herbizid, enthaltend einen für Herbizide üblichen Trägerstoff sowie ein Chinolinderivat der Formel

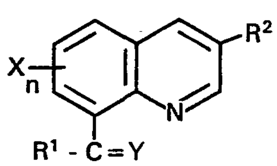
(I),

in der

X für Chlor in den Positionen 5 oder 7, wobei n 1 oder 2 bedeutet,

Y für Sauerstoff oder Schwefel,

$R^1$ für Halogen, die Gruppe $-NR^3R^4$, worin $R^3$ und $R^4$ gleich oder verschieden sind und Wasserstoff, $C_{1-6}$-Alkyl, $C_{2-6}$-Alkenyl, $C_{1-6}$-Hydroxyalkyl, Cyclohexyl oder Phenyl oder $R^3$ und $R^4$ zusammen einen Tetramethylen- oder Pentamethylenrest, wobei eine $CH_2$-Gruppe durch ein Sauerstoff- oder Stickstoffatom oder die $N(CH_3)$-Gruppe ersetzt sein kann, bedeuten oder für die Gruppe OM steht, worin M ein Äquivalent eines Alkalimetall- oder Erdalkalimetallions, Wasserstoff, $C_{1-8}$-Alkyl oder den Rest $H_2N^{\oplus}$-$R^3R^4$, in dem $R^3$ und $R^4$ die oben angegebenen Bedeutungen haben, bedeutet, und

$R^2$ für Wasserstoff, gegebenenfalls durch Halogen, Amino, Monoalkylamino, Dialkylamino oder Trialkylammonium mit 1 bis 4 Kohlenstoffatomen in einer Alkylgruppe, $C_{1-4}$-Alkoxy, $C_{1-4}$-Alkylthio, Trialkylphosphonium mit 1 bis 4 Kohlenstoffatomen in einer Alkylgruppe oder Triphenylphosphonium in ω-Stellung substituiertes $C_{1-6}$-Alkyl, für Formyl, Cyano, Carboxyl, Carbamoyl, N-Alkyl-carbamoyl oder N,N-Dialkyl-carbamoyl mit 1 bis 4 C-Atomen in einer Alkylgruppe oder für $C_{2-6}$-Alkenyl stehen oder in der der Rest - C - $R^1$ eine Nitrilgruppe oder
||
Y
eine Halogenmethylgruppe ist.

7. Herbizid, enthaltend inerte Zusatzstoffe und ein Chinolinderivat der Formel I gemäss Anspruch 6, wobei X Chlor in 7-Stellung, n 1, $R^1$ Wasserstoff oder die Gruppe OM, wobei M Wasserstoff, ein Äquivalent Alkalimetallion oder ein Dialkylammoniumion mit 1 bis 4 C-Atomen in einer Alkylgruppe bedeutet, $R^2$ $C_{1-4}$-Alkyl und Y Sauerstoff bedeuten.

8. Verfahren zur Bekämpfung unerwünschten Pflanzenwuchses, dadurch gekennzeichnet, dass man die Pflanzen und/oder ihren Standort mit einer herbizid wirksamen Menge eines Chinolinderivats der Formel I gemäss Anspruch 6 behandelt.

9. Verfahren zur Herstellung eines Herbizids gemäss Anspruch 7, dadurch gekennzeichnet, dass man inerte Zusatzstoffe mit 0,1 bis 95 Gew.-% eines Chinolinderivats der Formel I gemäss Anspruch 6 mischt.

**Claims**

1. A quinoline derivative of the formula

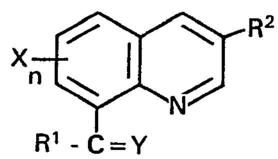
(I),

where

X is chlorine in the 5- or 7-position, n being 1 or 2,

Y is oxygen or sulfur,

$R^1$ is halogen or $-NR^3R^4$, where $R^3$ and $R^4$ are identical or different and are each hydrogen, $C_1$-$C_6$-alkyl, $C_2$-$C_6$-alkenyl, $C_1$-$C_6$-hydroxyalkyl, cyclohexyl or phenyl, or $R^3$ and $R^4$ together form a tetramethylene or pentamethylene radical, where one $CH_2$ group can be replaced by an oxygen or nitrogen atom, or $N(CH_3)$, or $R^1$ is OM, where M is one equivalent of an alkali metal or alkaline earth metal ion, hydrogen or $C_1$-$C_8$-alkyl, or is $H_2N^{\oplus}R^3R^4$, where $R^3$ and $R^4$ have the above meanings, and

$R^2$ is $C_1$-$C_6$-alkyl which is unsubstituted or substituted in the ω-position by halogen, amino, monoalkylamino, dialkylamino or trialkylammonium, where alkyl is of 1 to 4 carbon atoms, $C_1$-$C_4$-alkoxy, $C_1$-$C_4$-alkylthio, trialkylphosphonium, where alkyl is of 1 to 4 carbon atoms, or triphenylphosphonium, or

is formyl, cyano, carboxyl, carbamyl or N-alkylcarbamyl or N,N-dialkylcarbamyl, where alkyl is of 1 to 4 carbon atoms, or is $C_2$-$C_6$-alkenyl, or where the radical - C - $R^1$ is a nitrile or halomethyl group.
||
Y

2. A quinoline derivative of the formula I as claimed in claim 1, where X is chlorine in the 7-position, n is 1, $R^1$ is OM, M denoting hydrogen, one equivalent of an alkali metal ion or a dialkylammonium ion, where alkyl is of 1 to 4 carbon atoms, $R^2$ is $C_1$-$C_4$-alkyl and Y is oxygen.

3. A process for the preparation of a quinoline derivative of the formula I as claimed in claim 1, wherein a compound of the formula

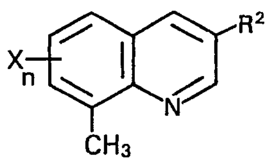 (II),

where X, n and $R^2$ have the meanings given in claim 1, is halogenated with a halogenating agent in the presence of a free-radical initiator at from 40 to 140°C and, if desired, the resulting compound of the formula

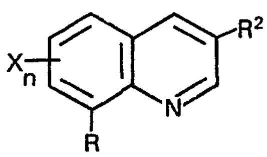 (III),

where X, n and $R^2$ have the meanings given in claim 1 and R is halomethyl, is converted into the compound of the formula I, where the radical - C - $R^1$
||
Y
is not halomethyl.

4. A process for the preparation of a quinoline derivative of the formula I, where $R^1$ is OM, M denoting hydrogen, Y denotes oxygen, and X, n and $R^2$ have the meanings given in claim 1, wherein a compound of the formula

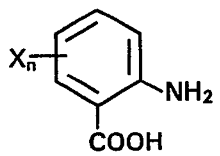 (IV),

where X and n have the meanings given in claim 1, is reacted with an aldehyde of the formula

$H_2C=C$ - CHO (V),
|
$R^2$

where $R^2$ has the meanings given in claim 1.

5. A process for the preparation of a quinoline derivative of the formula I, where X, n, $R^1$ and Y have the meanings given in claim 1 and $R^2$ is bromomethyl or dibromomethyl, wherein a compound of the formula

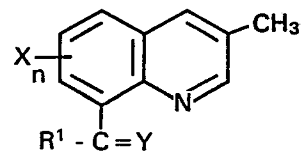 (VI),

where X, n, $R^1$ and Y have the meanings given in claim 1, is reacted with a bromine donor.

6. A herbicide containing a carrier usually used for herbicides and a quinoline derivative of the formula

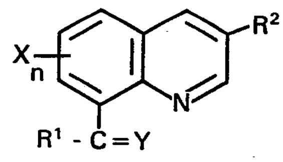 (I),

where

X is chlorine in the 5- or 7-position, n being 1 or 2,
Y is oxygen or sulfur,
$R^1$ is halogen or $-NR^3R^4$, where $R^3$ and $R^4$ are identical or different and are each hydrogen, $C_1$-$C_6$-alkyl, $C_2$-$C_6$-alkenyl, $C_1$-$C_6$-hydroxyalkyl, cyclohexyl or phenyl, or $R^3$ and $R^4$ together form a tetramethylene or pentamethylene radical, where one $CH_2$ group can be replaced by an oxygen or nitrogen atom, or $N(CH_3)$, or $R^1$ is OM, where M is one equivalent of an alkali metal or alkaline earth metal ion, hydrogen or $C_1$-$C_8$-alkyl, or is $H_2N^{\oplus}R^3R^4$, where $R^3$ and $R^4$ have the above meanings, and
$R^2$ is hydrogen, or $C_1$-$C_6$-alkyl which is unsubstituted or substituted in the ω-position by halogen, amino, monoalkylamino, dialkylamino or trialkylammonium, where alkyl is of 1 to 4 carbon atoms, $C_1$-$C_4$-alkoxy, $C_1$-$C_4$-alkylthio, trialkylphosphonium, where alkyl is of 1 to 4 carbon atoms, or triphenylphosphonium, or is formyl, cyano, carboxyl, carbamyl or N-alkylcarbamyl or N,N-dialkylcarbamyl, where alkyl is of 1 to 4 carbon atoms, or is $C_2$-$C_6$-alkenyl, or where the radical - C - $R^1$ is a ni-
||
Y
trile or halomethyl group.

7. A herbicide containing inert additives and a quinoline derivative of the formula I as claimed in claim 6, where X is chlorine in the 7-position, n is 1, $R^1$ is hydrogen or OM, M denoting hydrogen, one equivalent of an alkali metal ion or a dialkylammonium ion, where alkyl is of 1 to 4 carbon atoms, $R^2$ is $C_1$-$C_4$-alkyl and Y is oxygen.

8. A process for combating the growth of unwanted plants, wherein the plants and/or their location are treated with a herbicidally effective amount of a quinoline derivative of the formula I as claimed in claim 6.

9. A process for the production of a herbicide as claimed in claim 7, wherein inert additives are mixed with 0.1 to 95% by weight of a quinoline derivative of the formula I as claimed in claim 6.

**Revendications**

1. Dérivés de quinoléine de formule

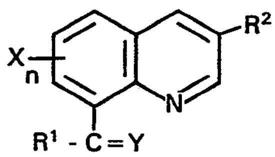

(I),

dans laquelle

X est mis pour chlore en positions 5 ou 7, n représentant 1 ou 2,

Y est mis pour oxygène ou soufre,

$R^1$ pour halogène, le groupe $-NR^3R^4$, où $R^3$ et $R^4$ sont identiques ou différents et représentent hydrogène, alkyle en $C_{1-6}$, alcényle en $C_{2-6}$, hydroxyalkyle en $C_{1-6}$, cyclohexyle ou phényle ou bien $R^3$ et $R^4$ ensemble représentent un reste tétraméthylène ou pentaméthylène, un groupe $CH_2$ pouvant être remplacé par un atome d'oxygène ou d'azote ou le groupe $N(CH_3)$, ou bien est mis pour le groupe OM, où M représente un équivalent d'un ion métal alcalin ou métal alcalino-terreux, hydrogène, alkyle en $C_{1-8}$ ou le reste $H_2N^{\oplus}R^3R^4$, dans lequel $R^3$ et $R^4$ ont les significations indiquées ci-dessus et,

$R^2$ est mis pour alkyle en $C_{1-6}$, éventuellement substitué en position ω par halogène, amino, monoalkylamino, dialkylamino ou trialkylammonium ayant 1 à 4 atomes de carbone dans un groupe alkyle, alcoxy en $C_{1-4}$, alkylthio en $C_{1-4}$, trialkylphosphonium ayant 1 à 4 atomes de carbone dans un groupe alkyle ou triphénylphosphonium, pour formyle, cyano, carboxyle, carbamoyle, N-alkyl-carbamoyle ou N,N-dialkyl-carbamoyle ayant 1 à 4 atomes de carbone dans un groupe alkyle ou pour alcényle en $C_{2-6}$ ou dans lequel le reste - C - $R^1$ est un groupe
||
Y
nitrile ou halogénométhyle.

2. Dérivés de quinoléine de formule I selon la revendication 1, caractérisé par le fait que X représente chlore en position 7, n représente 1, $R^1$ le groupe OM, M représentant hydrogène, un équivalent d'ion métal alcalin ou d'ion dialkylammonium à 1 à 4 atomes C dans un groupe alkyle, et $R^2$ représente alkyle en $C_{1-4}$ et Y oxygène.

3. Procédé de préparation de dérivés de quinoléine de formule I selon la revendication 1, caractérisé par le fait que l'on halogène, à une température comprise entre 40 et 140°C, en présence d'un inducteur de radicaux, un composé de formule

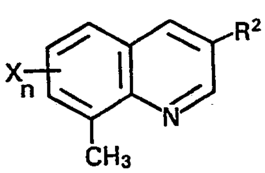

(II),

dans laquelle X, n et $R^2$ ont les significations indiquées dans la revendication 1, avec un agent halogénant, et l'on transforme éventuellement les composés ainsi obtenus de formule

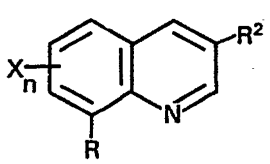

(III),

dans laquelle X, n et $R^2$ ont les significations indiquées dans la revendication 1 et R représente halogénométhyle, en le composé de formule I, dans lequel le reste -C - $R^1$ ne représente pas un groupe halo-
||
Y
génométhyle.

4. Procédé de préparation de dérivés de quinoléine de formule I dans laquelle $R^1$ est mis pour le groupe OM, M représentant hydrogène, et Y est mis pour oxygène, et X, n et $R^2$ ont les significations données dans la revendication 1, caractérisé par le fait que l'on fait réagir un composé de formule

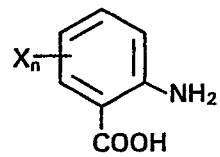

(IV),

dans laquelle X et n ont les significations indiquées dans la revendication 1, avec un aldéhyde de formule

$H_2C=C$ - CHO (V)
|
$R^2$

dans laquelle $R^2$ a les significations indiquées dans la revendication 1.

5. Procédé de préparation de dérivés de quinoléine de formule I dans laquelle X, n, $R^1$ et Y ont les significations indiquées dans la revendication 1 et $R^2$ est mis pour bromométhyle ou dibromométhyle, caractérisé par le fait que l'on fait réagir un composé de formule

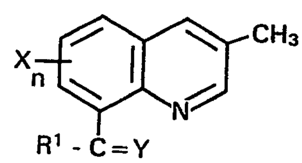

(VI),

dans laquelle X, n, $R^1$ et Y ont les significations indiquées dans la revendication 1, avec un agent délivrant du brome.

6. Herbicide contenant un support usuel pour herbicides, ainsi qu'un dérivé de quinoléine de formule

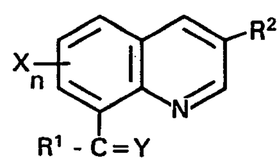

(I),

dans laquelle

X est mis pour chlore en positions 5 ou 7, n représentant 1 ou 2,

Y est mis pour oxygène ou soufre,

$R^1$ pour halogène, le groupe $-NR^3R^4$, où $R^3$ et $R^4$ sont identiques ou différents et représentent hydrogène, alkyle en $C_{1-6}$, alcényle en $C_{2-6}$, hydroxyalkyle en $C_{1-6}$, cyclohexyle ou phényle, ou bien $R^3$ et $R^4$ ensemble représentent un reste tétraméthylène ou pentaméthylène, un groupe $CH_2$ pouvant être remplacé par un atome d'oxygène ou d'azote ou le groupe $N(CH_3)$, ou bien est mis pour le groupe OM,

où M représente un équivalent d'un ion métal alcalin ou métal alcalino-terreux, hydrogène, alkyle en $C_{1-8}$ ou le reste $H_2N^{\oplus}R^3R^4$, dans lequel $R^3$ et $R^4$ ont les significations indiquées ci-dessus et,

$R^2$ est mis pour alkyle en $C_{1-6}$, éventuellement substitué en position ω par halogène, amino, monoalkylamino, dialkylamino ou trialkylammonium ayant 1 à 4 atomes de carbone dans un groupe alkyle, alcoxy en $C_{1-4}$, alkylthio en $C_{1-4}$, trialkylphosphonium ayant 1 à 4 atomes de carbone dans un groupe alkyle ou triphénylphosphonium, pour formyle, cyano, carboxyle, carbamoyle, N-alkyl-carbamoyle ou N,N-dialkyl-carbamoyle ayant 1 à 4 atomes de carbone dans un groupe alkyle ou pour alcényle en $C_{2-6}$ ou dans lequel le reste $-\underset{\underset{Y}{\|}}{C}-R^1$ est un groupe nitrile ou halogénométhyle.

7. Herbicide, contenant un additif inerte et un dérivé de quinoléine de formule I selon la revendication 6, où X représente chlore en position 7, n représente 1, $R^1$ le groupe OM, M représentant hydrogène, un équivalent d'ion métal alcalin ou d'ion dialkylammonium à 1 à 4 atomes C dans un groupe alkyle, et $R^2$ représente alkyle en $C_{1-4}$ et Y oxygène.

8. Procédé pour lutter contre la croissance de plantes indésirables, caractérisé par le fait que l'on traite les plantes et/ou leur biotope avec une quantité efficace comme herbicide d'un dérivé de quinoléine de formule I selon la revendication 6.

9. Procédé de préparation d'un herbicide selon la revendication 7, caractérisé par le fait que l'on mélange un additif inerte avec 0,1 à 95% en poids d'un dérivé de quinoléine de formule I selon la revendication 6.